# EUROPEAN PATENT APPLICATION

(11) **EP 4 064 294 A1**
(43) Date of publication of application: **28.09.2022**
(21) Application number: 22174859.3
(22) Date of filing: 23.05.2022
(51) Int. Cl.: G16H 50/20, G06N 3/00

(54) **ARTIFICIAL INTELLIGENCE-BASED SYSTEM FOR ANALYSING BIOMARKERS AND PERSONALISING CANCER TREATMENTS**

(30) Priority: 25.03.2021 ES 202130615 U
(71) Applicant: Keyzell Holding S.L., 41020 Sevilla (ES)
(72) Inventor: del Corral, Jose Esteban, Seville (ES); Solanes, Juan Antonio, Seville (ES)

(57) **Abstract**

The invention discloses an artificial intelligence system for analysing genetic and epigenetic markers, making diagnostics and recommending cancer personalized treatments. The system is divided into two modules: a module for treatment and analysis of the store data; and a digital platform for displaying the results.

## Description

### Technical field

The present invention is comprised in the field of oncology, and in particular, in a method assisted by neural networks (artificial intelligence), trained to analyse genetic biomarkers and produce a diagnostic value or a prognostic value to personalise a treatment or a combination of personalised drugs against cancer.

The system stores thousands of pieces of data in the form of datasets (cell lines, treatments and results), used for the purpose of generating predictions about custom treatments (synergy of several drugs) with a higher success rate for the tumour line and genetic and epigenetic biomarkers of each patient.

### State of the art

Cancer is still one of the main causes of morbimortality worldwide. The international Agency for Research has estimated that in the year 2020, 19.2 million cancers were diagnosed worldwide, with 10 million deaths. The same agency has estimated that about 30.2 million new cases will be diagnosed worldwide, with 16.3 million deaths in the year 2040. The cancers which cause a higher number of deaths are lung, liver, stomach, colon and breast cancer.

In this context, a report drawn up by the IQVIA Institute calculates that the global market of oncological therapeutic drugs will reach 200 billion dollars in 2022, with an average growth of 13 % in the next five years.

The first fundamental phase in the treatment of cancer is to establish a diagnosis based on a pathological examination. A series of tests must be performed to determine the extent of the tumour. Staging often requires substantial resources that can be prohibitively expensive in resource-poor settings where, due to late diagnosis, which in turn is a consequence of poor access to care, in most patients the disease is already in an advanced stage.

Once the diagnosis and extent of the tumour have been established to the extent possible, a decision has to be made on the most effective treatment in a given socio-economic setting.

This requires careful selection of one or more main treatment modalities; surgery, radiotherapy and systemic treatment, a choice that should be based on scientific evidence of the best available treatment taking into account available resources.

When the tumour is localised and small in size, surgery and sometimes radiotherapy alone are likely to achieve very good results. Chemotherapy alone can be effective for a small number of cancers, such as haematological malignancies (leukaemias and lymphomas), which can generally be considered to be widespread from the outset.

Nowadays, breakthroughs in technology and innovation increasingly allow for the early detection of certain diseases, even before the first symptoms develop, which makes it possible to activate the necessary action protocols to prevent the final onset of the pathology.

Document ES2436667 refers to biomarkers for diagnosing colorectal cancer.

This is made possible by biomarkers, a measurable and assessable physiological characteristic or change at the molecular, biochemical or cellular level that acts as an indicator of a normal or pathological biological process, or in response to a therapeutic intervention.

In fact, these indicators have become a key element in oncological treatments, as they allow the cellular nature of each tumour to be defined in order to know its possible evolution, and to apply, in each case, an individualised or personalised therapy.

Documents ES2537403 and ES 2611000 refer to the use of gene expression profiling to determine prognoses for cancer patients.

In this way, correctly identifying which patients are suitable for each treatment and developing techniques that provide valuable information on the evolution of a tumour process or the prognosis thereof are fundamental to improving the health of patients.

While the protocols followed in combination drug therapies are a wellestablished concept in cancer treatment, identifying the most suitable combination for a patient with a given genetic profile is challenging due to the size of the combinatorial space. Normally, this choice of guidelines and standards (treatment plan) is established across all hospitals on the basis of scientific and clinical experience, literature review and knowledge of previous protocols. In addition, indications and limitations are listed according to several tumour-related factors (type, location and size, involvement of lymph nodes or other organs) and patient-related factors (age, general state of health, comorbidities, predisposition of the patient), but without taking into account the genetics of the tumour and the patient in order to offer more personalised treatments.

It is known that cancer cells often have "alterations" in their genes, their proteins or their specific titular setting that distinguish them from normal cells, so one of the main treatment routes consists of using drugs specifically designed to target cancer cells, blocking their growth and spread, without affecting normal cells. This is what is known as targeted therapy. This type of therapy allows for personalised or precision medicine as it is designed to specifically target certain changes or substances in cancer cells. This target may be different even among subjects with the same type of cancer. Hence the great variability and combinatorial analysis that exists, which makes it necessary to have tools that facilitate the joint analysis of all the cases of cancer in order to find the most effective treatment. Finding that specific target molecule (identification of genes, proteins or other tumour factors) makes the identification of patients with treatment more precise or personalised whenever possible.

A scientific team from the University of Granada and the Genomics Centre for Oncological Research has developed a technique that can identify the cells causing metastasis by means of a blood test. With the new biomarkers, all existing subpopulations of circulating tumour cells can be identified for the first time.

The American Society of Geneticists and Molecular Biologists (ASCO) has published new clinical practice guidelines on the use of biomarkers to guide decisions about therapies in early-stage breast cancer.

In the publication "Biomarkers in cancer, contribution of epigenetics in personalised medicine", the fundamental role of genetics and epigenetics in the search for personalised therapies in cancer treatment, as well as prognostic markers of cancer phenotypes, are presented.

The number of biomarkers identified makes a myriad of variables difficult or impossible for medical teams to relate these variables to achieve a desired and effective predictive prediction. Consequently, what is needed is an approach to diagnose and provide personalised treatments that incorporates a system capable of accommodating a large number of factors, e.g. genetic and epigenetic factors, and for this there are neural networks capable of working with high volumes of patient and tumour data, to rapidly process the data and obtain a diagnosis and forecast.

Now that the use of Neural Networks or Artificial Intelligence systems has begun in the oncology sector, researchers at Tomsk State University have developed a new method for diagnosing adenocarcinoma, a malignant tumour of the prostate gland, which uses artificial intelligence to identify whether a tumour is present and determine the stage of the disease. According to one of the researchers, artificial intelligence not only detects the presence of cancer cells with one hundred percent accuracy, in the researchers' own words, but also evaluates the tumour according to the Gleason scale, which marks the severity and progression of the cancer and is important in terms of predicting the course of the disease.

The journal Nature published a study revealing that Google has developed an algorithm that reduces false positives, that is, people who are diagnosed with cancer when they do not actually have it, by 3.5%, and false negatives, i.e., those cases in which cancer is not detected but is actually present, by 8.1%.

Nataša Pržulj, researcher at the Barcelona Supercomputing Center (BSC), has led the creation of a new artificial intelligence-based computational method which accelerates the identification of new cancer-related genes. The method and its results have been published in Nature Communications.

Computational approaches to finding treatments as learning models to fight cancer have been one of the most time- and cost-efficient ways in recent years, as they allow hidden patterns to be found and complex tasks that often take months to be performed in a matter of seconds. These approaches rely especially on the efficient analysis of a large amount of data (high dimensionality) in order to try to explain the variability that exists in a disease as heterogeneous as cancer. In this aspect, Deep Learning has proven to have a great impact on this type of complex pattern search tasks (abstract representations), given that it is perfectly adapted to this type of challenges as a result of the high throughput of its models to extract important characteristics and it prediction capacity from large datasets.

### Explanation of the invention

An objective of the present invention relates to disclosing a novel method based on deep learning from data, taking into account relevant aspects such as: The peculiar characteristics of the cancer of each patient. There is enormous variability in the cancer. This variability manifests itself at four levels: 1.- within the same cancer there are areas that are different from one another; 2.- between each type of cancer; 3.- in the same type of cancer between different people; and 4.- in the same cancer and in the same person a change in its characteristics throughout the evolution thereof takes place.

The peculiar characteristics of the person having the cancer (Host). Each person is different, and this difference is determined by the genetic information that he or she carries, which conditions and directs the functioning of his or her body. These differences obviously also condition key elements in the progression of cancer, such as the body's response to the disease, the absorption and distribution of drugs and the onset of toxicity (damage to non-cancerous cells), which translates into undesirable effects (manifestation in the organs of the damage caused by the drugs in non-cancerous cells).

The known effect of the anti-cancer drugs. Each drug is expected to have a different effect on each type of cancer. This effect is determined by the following aspects: the peculiar characteristics of the drug, the peculiar characteristics of the specific cancer and the peculiar characteristics of the host in which this cancer resides. It is known that for some cancers, treatment with a single drug is effective, but clinical experience has shown that in most cases of cancer, the combined use of two or three drugs is more effective. This is based on the variability of cancer, so that in the same cancer and in the same person there may be areas with different characteristics that condition the sensitivity to a drug, so that using several drugs increases the possibilities of "covering" all the areas with different peculiarities of the same cancer.

The present invention uses genomic information, the natural and clinical history of each patient with cancer as input information. By incorporating genomic information, it learns to distinguish different cancer cell lines and find which specific drug combinations used in clinical practice have maximum efficacy in a given cell line. Internally, it uses a normalisation strategy to minimise the heterogeneity of the input data and combines information about the cancer cell line and the set of drugs in its hidden neural network layers to form a composite representation of the model that eventually leads to precise predictions of possible treatments suitable for the characteristics of the input cancer subject, with their corresponding doses. This thus constitutes a Clinical Decision Support System, capable of interpreting information from large amounts of data (functional genomics).

These treatments provided consist of combinations of drugs, as it has been shown that such therapies tend to have greater efficacy, lower toxicity and reduced drug resistance compared to monotherapy. To this end, the invention is capable of analysing the particular peculiarities of a given person's cancer, in the particular context of that person as a host interacting with that cancer with a dynamic system, taking into account the variability of the biological system. Precisely the main problem which causes cancer to still be a deadly disease is its variable and dynamic nature, which gives it the ability to adapt to the environment (pressure from interaction with the host) and to the targeting by available drugs, and therefore an effective mechanism of "escape" and progression. The only way to combat it is by controlling that variability and predicting that dynamism (which gives it capacity of resistance and progression). It is therefore important to find the combination of drugs that is best suited to a given subject by increasing their survival free of cancer progression.

In this sense, the variability of cancer and its forms of evolution are explored, so that in each case it will be possible to anticipate the mechanisms of adaptation and evolution (escape) of each case of cancer by designing the optimal treatment effective at time "0" and in any scenario of evolution (escape) thereof. In other words, cancer is not only treated on the basis of the characteristics present at the time of diagnosis, but also on the basis of the foreseeable characteristics it would have in each of the possible escape scenarios for that particular cancer.

The development of the invention consists of the construction of a model according to the claims in which two main phases are distinguished: on one hand, the search for patterns and learning, and on the other, the prediction of the treatment of a subject.

For a first model training approximation, a dataset with 18,458 cell line samples was used, where each sample consists of tumour characteristics (location, type, species) and genetic and immunohistochemical profile of the cell lines. The dataset covers 527 different combinations of drugs (treatments), each tested on 46 cancer cell lines.

Despite the importance of cancer cell lines in biomedical research, their ability to accurately represent the *in vivo* state is often questioned. The reason is that even if there is a high genomic correlation between the original tumour and the derived cancer cell line, it is still far from perfect. However, using cell lines in the first training phases allows the acquisition of a first structure very close to that expected *in vitro,* as the first layers begin to learn the most basic and common characteristics shared between both experiments, so that the large amount of information and knowledge acquired related to the resolution of the problem in statistical terms is taken advantage of and used. A technique widely used in Deep Learning, called Transfer Learning, is very useful in this case as it is required to address the same problem.

Clearly, *in vivo* information is required to thus validate and evaluate the performance of accurate pre-trained models *in vitro,* but these trained and validated models can thus be used to recognise the desired characteristics with pattern modification or modification and addition of subsequent layers, without having to be trained from scratch, until acceptable performance is achieved. It is the latter layers that specialise in solving the specific problem *in vivo,* while the first deep layers are those that explain the more general aspects both *in vitro* and *in vivo.*

In this way, reliable predictions provide a valid guide for *in vitro* and *in vivo* research. In addition, the methods developed to use genomic information for their predictions offer the opportunity to apply them also in an *in vivo* setting. These predictive models are therefore a major step towards precision medicine. Cancer is a difficult disease to treat, so having as much knowledge as possible at all levels and from different perspectives allows there to be greater inference capacity in the fight against cancer.

With the necessary information available, supervised training is carried out as the response of these cell lines under consideration to the various drug combinations is known a priori, going through the thousands of possibilities. The purpose of the training is precisely the medical decision algorithm: better understanding of disease mechanisms and identification of potential treatment candidates.

### Brief description of the drawings

To better understand the present invention, a series of figures is attached by way of a non-limiting example is attached to summarise the working of the present invention.

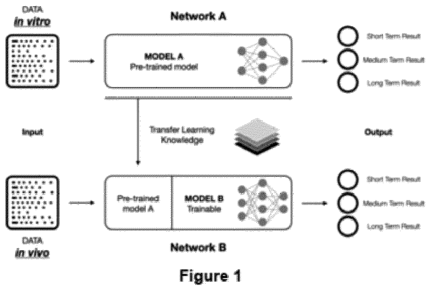

Figure 1 depicts the training model according to the features of the invention.

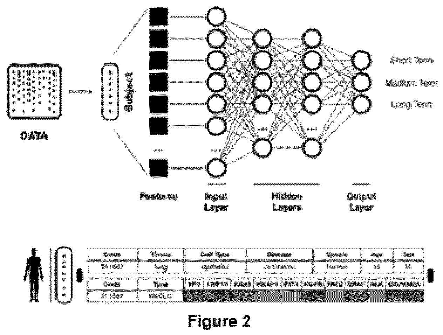

Figure 2 depicts the data analysis model according to the features of the invention.

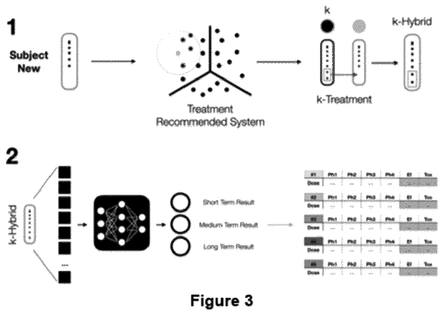

Figure 3 depicts the result model according to the features of the invention.

### Detailed disclosure of an embodiment of the invention.

One of the best ways to approach the problem, taking into account the data typology and characteristics, is to generate a model capable of mapping the input vectors representing the samples to 3 output values: "Short, Medium, Long Term Result".

The model is characterised by the use of deep neural networks. In this case, it specifically consists of a Feed Forward Neural Network. Said networks are made up of a superposition of layers forming what is known as a multilayer perceptron capable of solving the problem in question. Thus, in this type of architecture, 3 types of layers are distinguished: input layer, hidden layers and output layer. The main objective of using this perspective is to obtain an accurate prediction model by configuring the assignment patterns of an input A (set of subjects with their respective individual characteristics) to a given output B (response of a subject to a given combination of drugs). In this case, it is supervised learning because it is based on an established dataset, and the response of the data is known, i.e., the outputs (B) that define them are known. The model will ultimately match the attributes of the modelled data to the labels they possess, extracting correlation information between variables. Hence, it is important that the data available for training are exposed in the best possible way, without mislabelling and trying to make all the input features available to the model.

During the training step, the data available in the data repository is used. In the first phases, this data will be only that data collected during the studies carried out previously, but they will be complemented by the data predicted by the tool. In this way, each of the subjects will be represented by a vector of N inputs constructed from the individual characteristics of the subject and his or her cancer. Within these individual input properties, the following are distinguished: individual characteristics (such as age, sex, tumour location), gene expression values (DNA, RNA, immunohistochemistry) and treatment used on the subject. This vector constitutes the individual input of the model (input layer), so its length corresponds to the number of neurons present in this layer.

As for its output layer, it is made up of 3 neurons that correspond to "Short, Medium, Long Term Result". This type of result quantifies both the effectiveness and toxicity of the treatment used, with a value between 0 and 150 indicating the degree of cell survival. The aim is always to ensure that the system provides the optimal result. Optimal is understood as allowing a higher efficacy (lower mean value) when it is one of the tumour cell lines and lower toxicity (higher mean value) when it is a normal cell line.

Finally, with regard to the hidden layers, a conservative methodology will be chosen, avoiding underfitting and overfitting, i.e., not being able to learn or losing generalisation capacity, respectively. The starting point is a simple structure to which a larger number of neurons, and perhaps even a layer, will be added as better results and greater precision are sought, and with the transition of data from *in vitro* to *in vivo.* The primary structure used with training given the dimensions of the problem is to have two hidden layers with 8192 and 4096 neurons, respectively. The structure of the network is pyramidal, wherein the input information is propagated through the different layers of the network until the desired quality is provided.

Some technical aspects necessary for the correct functioning and performance of the model: the normalisation of quantitative data where all inputs are standardised with mean 0 and variance 1, and the hyperbolic tangent is applied. Qualitative, and therefore categorical, variables are binarised by means of One Hot Encodering techniques. The hidden layers apply rectified linear activations, and the output layer uses a linear activation. The objective function that is minimised during training is the mean square error.

One of the most important considerations to take into account is the fact that the model should only provide those treatments the drug combinations of which have already been tested, i.e., they have already been used on some of the subjects being trained and are available in the database. This fact greatly reduces the number of combinations that can be achieved with the drugs studied, and determines the way in which the model is constructed. In principle, the model should not provide an untested combination of drugs, since no studies of subjects' behaviour towards said "new combination" are available. Thus, only treatments that have already been used in studies will be considered.

With the trained model, when a patient is entered with a specific treatment, it provides short, medium and long term results, using the patterns learned during the training phase. The production step is now distinguished.

In this step, the data input includes two aspects: the patient's tumour type (which can be one of those included in the database or another not yet included) and the genetic profile of the tumour which has two types of DNA profile (DNA Hotspot genes) and RNA profile (RNA Fusion Drivers). The type of tumour of the "test" subject will be known. And all "test" patient cases will undergo a DNA / RNA study similar to the one performed on the cell lines / cases included in the database in which the treatment applied and the possible outcome obtained were known during the training phase. Thus, a prediction is made based on the similarity of the patient's DNA / RNA profile to the DNA / RNA profile of the cases included in the database, of the different treatment options and the potential effect of each of them.

This step in turn consists of a two-phase algorithm. The first is the identification of possible treatments and the second is the evaluation of the treatments considered.

Focusing on the first phase of the algorithm, it will be in charge of determining which pharmacological treatment options might best suit the subject. A treatment recommendation system is used for this purpose. This algorithm uses a similarity measurement (cosine distance) between the input subject and the subjects already present in the database, which have a treatment already assigned to them. With this measurement, the system knows the spatial distance between two subjects and therefore quantifies how close they are and therefore how similar they are. Starting from those with the highest similarity (k neighbours), the treatment used (k treatments) is extracted, and this is added to the new subject information.

All the combined information, both the new subject's own characteristics (age, sex, genetic and immunohistochemical profiles) and a possible treatment, are concatenated and constitute the input to the neural network model. This entire workflow is performed for each of the k-neighbours considered.

In the second phase, the model will evaluate the subject's characteristics with the treatment by providing the potential efficacy of said drug combination on the subject, which is represented by the model output.

The ideal treatments are those that achieve maximum efficacy with minimum (if possible "zero") toxicity.

Finally, a list of all possible treatment options is obtained, classified from optimal to sub-optimal treatment option for a "test" subject.

The tool is intended to increase the efficiency, productivity and clinical confidence of system operators, reducing the high variability and burden on their workload. This results in an intelligent, automated workflow that provides skillful processing and interpretation of information, allowing data to be converted into processable information to extract the medical oncology knowledge needed to of the most suitable cancer treatment, in the right order, at the right time.

## Claims

1. An artificial intelligence-based system for the analysis of genetic and epigenetic biomarkers, diagnosis and recommendation of personalised cancer treatments, a process known as precision medicine, **CHARACTERISED by** being divided into the following modules:
a) Stored data processing and analysis module.
b) Digital platform for viewing the results.

2. The module according to claim 1, **CHARACTERISED by** storing thousands of pieces of data in the form of datasets (cell lines, genetic and epigenetic biomarkers, treatments and results), used for the purpose of generating predictions in real time about custom treatments (synergy of several drugs) with a higher success rate for the tumour line and biomarkers characteristic of a patient.

3. The system according to claims 1 and 2, **CHARACTERISED by** a subsystem of neural networks (machine learning, artificial intelligence) relating to the processing and analysis of all the information about genetic and epigenetic biomarkers, recorded in the form of datasets.

4. The system according to claim 1 **CHARACTERISED by** the use of an algorithm to predict personalised treatments against tumour lines.

5. The system according to claims 1, 3 and 4, **CHARACTERISED in that** the user can access an interface with statistical information, a diagnostic report and treatment recommendations.
